# EUROPÄISCHE PATENTANMELDUNG

(11) **EP 2 987 535 A1**
(43) Veröffentlichungstag der Anmeldung: **24.02.2016**
(21) Anmeldenummer: 15180561.1
(22) Anmeldetag: 11.08.2015
(51) Int. Cl.: A61Q 19/08, A61K 8/98, A61K 8/35, A61K 8/73, A61K 8/25

(54) **WIRKSTOFFSYSTEM**

(30) Priorität: 18.08.2014 DE 102014111768
(71) Anmelder: Hönscher-Sickert, Monika, 46286 Dorsten (DE)
(72) Erfinder: Hönscher-Sickert, Monika, 46286 Dorsten (DE)
(74) Vertreter: Stenger Watzke Ring

(57) **Zusammenfassung**

Die Erfindung betrifft ein Wirkstoffsystem für kosmetische Zwecke zur topischen Anwendung auf der Haut, aufweisend einen Wirkstoffträger und einen Wirkstoffkomplex, dadurch gekennzeichnet, dass der Wirkstoffkomplex für kosmetische Zwecke zur topischen Anwendung auf der Haut, aufweisend einen Wirkstoffträger und einen Wirkstoffkompiex, dadurch gekennzeichnet, dass der Wirkstoffkomplex wenigstens eine in der Natur vorkommende, nicht synthetische Nährstoffquelle enthält. Ferner betrifft die Erfindung ein kosmetisches Verfahren zur Faltenreduzierung, wobei das erfindungsgemäße Wirkstoffsystem bei einer Einzelanwendung auf die Haut aufgebracht wird, wobei eine Mehrzahl von Einzelanwendungen durchgeführt wird, wobei der Zeitpunkt der jeweiligen Einzelanwendung durch eine individuell erstellbare Anwendungssequenz bestimmt wird, wobei die Anwendungssequenz aus wenigstens zwei chronologisch aufeinander folgenden Anwendungsphasen mit anwendungsphasenspezifischen Einzelanwendungsfrequenzen gebildet wird, wobei den Anwendungsphase jeweils eine voneinander verschiedene Einzelanwendungsfrequenz zugewiesen wird.

## Beschreibung

Die Erfindung betrifft ein Wirkstoffsystem für kosmetische Zwecke zur topischen Anwendung, aufweisend einen Wirkstoffträger und einen Wirkstoffkomplex.

Unsere Haut besteht im Wesentlichen aus drei Hautschichten, der Unterhaut (Subcutis), der Lederhaut (Dermis) sowie der Oberhaut (Epidermis). Verantwortlich für ein jugendliches, faltenfreies Erscheinungsbild zeichnet insbesondere die Dermis. Diese besteht zum überwiegenden Teil aus Bindegewebszellen (Fibroblasten) sowie den von den Fibroblasten mittels Proteinbiosynthese hergestellten Bindegewebsfasern Kollagen und Elastin.

In jungen Jahren ist unsere Haut einem ständigen Erneuerungsprozess unterworfen, um alte, verbrauchte Fibroblasten durch neue zu ersetzen und/oder den durch Wachstum bedingten erhöhten Zellbedarf auszugleichen. Mit fortschreitendem Lebensalter unterliegen die Fibroblasten jedoch einer teilweise umweltbedingten, teilweise genetisch vorgesehenen Alterung, die insbesondere mit einer sukzessiven Verringerung der Zellteilungsrate einhergeht. Die beiden hierfür verantwortlichen biologischen Prozesse sind zum einen der programmierte Zelltod (Apoptose), wodurch schadhafte Zellen aus dem Organismus entfernt werden, und zum anderen die Zellseneszenz, die nach einer genetisch vorbestimmten Anzahl von Zellteilungen die Zelle mitotisch inaktiviert, wodurch sich diese nicht mehr teilen kann. Infolge der stetig abnehmenden Zellteilungsrate stehen im Alter immer weniger Zellen zur Produktion der Bindegewebsfasern zur Verfügung, wodurch das Unterhautfettgewebe zunehmend dünner wird und die Haut weniger Fett und Wasser speichern kann. Im Ergebnis stehen zum Ärgernis des überwiegenden Teils der Menschheit unerwünschte Falten, insbesondere im Gesicht, die mit fortschreitendem Lebensalter das Aussehen und die Selbstwahrnehmung immer stärker negativ beeinflussen.

Hinzu kommt ein weiterer Aspekt. Während die Haut im jugendlichen Alter noch verhältnismäßig ausreichend mit den, für die Leistungsfähigkeit der Zellen notwendigen, Nährstoffen durch den Blutkreislauf versorgt wird, nimmt die Durchblutung der Haut im Alter ab. Die Hautzellen leiden infolgedessen an einem stetigen Mangel an Nährstoffen, wodurch insbesondere die Synthese von Kollagen und Elastin gehemmt wird. Es wird daher mit fortschreitendem Lebensalter zunehmend wichtig, die noch verbliebenen, zur Bindegewebsfaserbildung fähigen Fibroblasten mit den benötigten Nährstoffen zu versorgen.

Eine Fülle von Nahrungsergänzungsmittel sind zu diesem Zweck aus dem Stand der Technik bekannt, deren Nutzen allerdings oftmals zweifelhaft ist. Insbesondere, da sie das Problem der mangelnden Zuleitung der Nährstoffe durch verringerte Durchblutung nicht zu beheben vermögen. Aus dem Stand der Technik haben sich daher Präparate zur topischen Anwendung direkt auf der Haut etabliert, die die Nährstoffe transdermal zu den Zellen transportieren, wodurch das Problem einer mangelnden Durchblutung der Haut umgangen wird. Diesbezüglich ist aus dem Stand der Technik insbesondere die DE 10340684 A1 bekannt geworden.

Sie beschreibt eine kosmetische oder pharmazeutische Zusammensetzung, die insbesondere durch Zuführung des Vitamins B6 die Kollagenexpression der Fibroblasten erhöht. Zusätzlich zu Vitamin B₆ können weitere Vitamine, Provitamine, Aminosäuren und weitere Inhaltsstoffe Bestandteil der Zusammensetzung sein. Nachteilig ist hierbei, dass durch die künstlich gesteigerte Expression von Kollagen der Energieverbrauch der Zelle enorm erhöht wird. Hierdurch wird eine vorübergehende Steigerung der Leistungsfähigkeit der Zelle mit einer verkürzten Gesamtlebensdauer der Zelle erkauft, so dass besagte Zusammensetzung zwar kurzfristig eine Minderung der Falten bewirkt, aber einer langfristigen und nachhaltigen Faltenreduktion abträglich ist. Mit zunehmendem Alter ist dies besonders problematisch, da verlorene Zellen nicht ohne weiteres ersetzt werden können.

Ferner ist es bei dem Stand der Technik notwendig, die einzelnen Bestandteile der dort offenbarten Zusammensetzung einzeln zu synthetisieren, was mit einem erheblichen Aufwand verbunden ist. Ferner fallen bei einer solchen Synthese unerwünschte Nebenprodukte an, die aufwendig entsorgt werden müssen bzw. in der Zusammensetzung als Verunreinigung verbleiben, aber keinen Beitrag zur Faltenreduzierung leisten.

Der Erfindung liegt daher die Aufgabe zugrunde, ein Wirkstoffsystem bereitzustellen, welches Faltenbildung nachhaltig, langfristig und auf natürlichem Wege mindert.

Zur Lösung der Aufgabe wird ein eingangs genanntes Wirkstoffsystem vorgeschlagen, welches sich dadurch auszeichnet, dass der Wirkstoffkomplex wenigstens eine in der Natur vorkommende, nicht-synthetische Nährstoffquelle enthält.

Der erfindungsgemäße Vorteil der Nutzung einer natürlich vorkommenden Nährstoffquelle liegt in der Bereitstellung eines synergetisch wirkenden Nährstoffverbundes aus einer Vielzahl von Nährstoffen. Es hat sich überraschend gezeigt, dass die von der Natur vorgesehenen Bindungen zwischen den einzelnen Nährstoffen sowohl einen Transport durch die Epidermis fördert und darüber hinaus in ihrer ganzheitlichen Wirkung auf die biochemischen Vorgänge in den Hautzellen synthetischen Nährstoffen, die isoliert nebeneinander eine Zusammensetzung bilden weit überlegen ist. So vermag die erfindungsgemäße natürliche Nährstoffquelle sowohl die Energiegewinnung der Zelle als auch die Proteinbiosynthese der Bindegewebsproteine Kollagen und Elastin in synergetischer Weise zu verbessern. Ein Ungleichgewicht zwischen forcierter Kollagenexpression und unzureichender Energieversorgung der Zelle kann somit vorteilhafterweise vermieden werden.

Gemäß einer bevorzugten Ausgestaltung der Erfindung ist die erfindungsgemäße Nährstoffquelle aus Eiklar gebildet. Eiklar im Sinne der Erfindung bezeichnet hierbei die den Dotter der Eier von insbesondere Reptilien oder Vögeln umgebende Mischung aus dünnflüssigen und gallertartigen Bestandteilen. Eiklar kann hierbei in nativer Form oder in getrockneter Form, insbesondere als Pulver oder Granulat, genutzt werden.

Es hat sich überraschend gezeigt, dass Eiklar einen nie gekannten, positiven Effekt auf die Faltenbildung der Haut hat. Falten können langfristig gemindert und das Hautbild insgesamt verjüngt werden. Es wird anmelderseitig davon ausgegangen, dass die spezielle Kombination von im Eiklar enthaltenen Nährstoffen für diesen Effekt verantwortlich ist. Eiklar von insbesondere Hühnereiern besteht aus 88 % Wasser, etwa 40 verschiedenen Proteinen und Glycoproteinen (11%), Kohlenhydraten in Form der Glycoproteine, Mineralstoffen und Spurenelementen, wie Natriumionen, Kaliumionen, Calciumionen, gebundenem Phosphor und gebundenem Eisen, sowie aus den Vitaminen A, B1, B2, C und Niacin. Von den ca. 40 im Eiklar enthaltenen Proteinen macht das Ovalbumin mit 58 % den größten Teil aus. Weitere Proteine sind insbesondere das Ovotransferrin (13 %), das Ovomucoid (11 %), die Muramidase (3,5 %) sowie die G2- und G3-Globuline (4 %).

Es hat sich gezeigt, dass die im Eiklar enthaltenen Wirkstoffe in synergetischer und nachhaltiger Weise die Produktion von Kollagen und Elastin in den Fibroblasten unterstützt, indem es sowohl die benötigten Bausteine für die Proteinbiosynthese der vorgenannten Proteine bereitstellt, als auch die hierfür benötigte Energie, durch Bereitstellung von energiereichen Kohlenhydraten sowie von Phosphor und Eisen als Bestandteil der Atmungskette, in Form von ATP (Adenosintriphosphat) liefert. Die im Eiklar enthaltenen Proteine erfüllen hierbei eine Doppelaufgabe, indem sie einerseits selbst als Aminosäurequelle für die Proteinbiosynthese dienen und andererseits als transdermales Transportmedium für die übrigen Nährstoffe fungieren. Für die Proteinbiosynthese von Kollagen fungiert Ovalbumin insbesondere als Quelle für die Aminosäuren Prolin- und Glycin, welche im Kollagen zu einem erheblichen Teil vorhanden und für dessen Funktionalität unentbehrlich sind. Auch zur Elastinbiosynthese, welche erhebliche Mengen der Aminosäure Valin benötigt, leistet das Valin-reiche Ovalbumin einen wertvollen Beitrag. Darüber hinaus ist Ovalbumin äußerst reich an der Aminosäure Serin, welche ein wichtiger Bestandteil der für die Proteinbiosynthese benötigten SR-Proteine ist.

Es hat sich überraschend gezeigt, dass die Eiklarproteine in vorteilhafter Weise besonders epidermisgängig sind und darüber hinaus den Transport der übrigen Nährstoffe zu den in der Dermis befindlichen Fibroblasten unterstützen. So liefern insbesondere die Glycoproteine Ovalbumin, Ovotransferrin und Ovomucoid die für die ATP Gewinnung benötigten Kohlenhydrate. Die für die ATP Gewinnung essentiellen Spurelemente Phosphor und Eisen werden ebenfalls von Eiklarproteinen durch die Epidermis transportiert. So liegt Phosphor in veresterter Form an den Serineinheiten des Ovalbumin vor, während Eisen von Ovotransferrin komplexiert wird.

Auch die im Eiklar enthaltenen Vitamine leisten einen wichtigen Beitrag zur Faltenreduktion. So bewirkt Vitamin A, auch bekannt als Retinol, eine Erhöhung der Kollagenproduktion in den Fibroblasten und unterstützt die Bildung neuer Blutzellen in der Haut. Vitamin C (Ascorbinsäure) ist ein starkes Anti-Oxidant, welches die Hautzellen vor Schäden durch freie Radikale schützt und so einen wichtigen Beitrag zu deren Leistungsvermögen liefert. Überdies ist Vitamin C als enzymatischer Cofaktor ebenfalls an der Kollagensynthese beteiligt. Vitamin B1 (Thiamin) bildet als Cofaktor einen Bestandteil des Enzyms Pyruvatdehydrogenase, welche bei der Pyruvatdecarboxylierung als Teil der Atmungskette die Reaktion von Pyruvat zu Acetyl-CoA (Acetyl-Coenzym A) katalysiert. Vitamin B2 (Riboflavin) spielt eine Schlüsselrolle in der Atmungskette des Menschen, da es unter anderem essentieller Bestandteil der an der oxidativen Phosphorylierung und der Pyruvatdecarboxylierung beteiligten Flavin-Cofaktoren ist. Niacin (veraltet: Vitamin B3) ist als Edukt für die Synthese der Cofaktoren NAD (Nikotinamid-Adenin-Dinukleotid) und NADP (Nikotinamid-Adenin-Dinukleotid-Phosphat), welche sämtliche im Organismus ablaufenden Redoxprozesse, so auch in der Atmungskette, katalysieren, für den Körper von elementarer Bedeutung.

Auf diese Weise verbessern die im Eiklar enthaltenen Wirkstoffe in synergetischer Weise die Nährstoffversorgung der Haut, insbesondere der Fibroblasten, sowohl hinsichtlich der Proteinbiosynthese der Bindegewebsproteine Kollagen und Elastin, als auch hinsichtlich der ATP (Energie) Gewinnung. Der Haut wird somit in die Lage versetzt, langfristig Bindegewebsfasern zu bilden, wodurch die Bildung von Falten vermindert wird. Ferner ist das Eiklar eine kostengünstige, leicht zugängliche Quelle der vorgenannten Wirkstoffe, die ansonsten alle mit hohem Aufwand einzeln synthetisiert oder extrahiert werden müssten.

Der Wirkstoffträger dient der Aufnahme des Wirkstoffkomplexes und Verbesserung der topischen Anwendbarkeit des Wirkstoffsystems. Er ist vorzugsweise als Flüssigkeit oder Gel ausgebildet. Hierdurch ist die besonders homogene Verteilung des Wirkstoffkomplexes im Wirkstoffträger möglich, wodurch vorteilhafterweise eine gleichmäßige Einwirkung aller Wirkstoffe über den gesamten räumlichen Anwendungsbereich gewährleistet ist.

Gemäß einer bevorzugten Ausgestaltung der Erfindung enthält der Wirkstoffkomplex ferner Kieselerde. Besonders bevorzugt liegen Eiklar und Kieselerde in einem Massenverhältnis von 3:1 bis 1:1 und weiter bevorzugt in einem Massenverhältnis von 1:1 im Wirkstoffkomplex vor. Es hat sich gezeigt, dass Kieselerde insbesondere in Kombination mit Eiklar zu einer weiteren Verbesserung des Faltenbildes führt. Darüber hinaus führt Kieselerde in Verbindung mit Eiklar zu einem beschleunigten transdermalen Transport durch die Epidermis.

Gemäß einer weiteren bevorzugten Ausgestaltung der Erfindung enthält der Wirkstoffkomplex Ubichinon-10 (Q10). Ubichinon-10 ist durch seine katalysierende Wirkung maßgeblich an dem, der ATP Gewinnung zugrunde liegenden biochemischen Prozess der oxidativen Phosphorylierung beteiligt. Vorteilhafterweise bewirkt Ubichinon-10 als Bestandteil des Wirkstoffkomplexes insbesondere in Verbindung mit Eiklar und Kieselerde eine weitere Verbesserung des Faltenbildes. Anmelderseitig wird davon ausgegangen, dass die im Wirkstoffkomplex enthaltenen Kohlenhydrate, das gebundene Eisen, das gebundene Phosphor und Ubichinon in synergetischer Weise die Proteinbiosynthese der Bindegewebsproteine energetisch fördern, indem sie die Voraussetzung für eine erfolgreiche ATP-Synthese schaffen.

Gemäß einer bevorzugten Ausgestaltung der Erfindung enthält das Eiklar wenigstens zum Teil Proteinhydrolysate der in ihm enthaltenen Proteine. In Proteinhydrolysaten sind die Peptidbindungen der Aminosäuren durch Hydrolyse gespalten, weshalb die Aminosäuren in ungebundener Form vorliegen. Vorteilhafterweise kann somit die Bioverfügbarkeit einzelner Aminosäuren verbessert werden, wodurch die Proteinbiosynthese von Kollagen und Elastin beschleunigt wird.

Gemäß einer bevorzugten Ausgestaltung der Erfindung enthält der Wirkstoffträger cyclische Oligosaccharide, besonders bevorzugt Cyclodextrine. Cyclodextrine bewirken vorteilhafterweise eine Verbesserung der Epidermisgängigkeit des Wirkstoffkomplexes. Insbesondere erhöhen Cyclodextrine durch Ausbildung eines Gast-Wirt-Komplexes mit den im Eiklar enthaltenen Vitaminen und mit bevorzugt enthaltenen Proteinhydrolysaten eine Verbesserung der Epidermisgängigkeit der Einzelkomponenten, wodurch die Nährstoffzufuhr insgesamt verbessert wird.

Es ist ferner eine Aufgabe der Erfindung, ein Verfahren zur Herstellung des erfindungsgemäßen Wirkstoffsystems vorzuschlagen, welches hinsichtlich seiner Verfahrensführung gegenüber dem Stand der Technik vereinfacht ist.

Zur Lösung dieser Aufgabe wird ein Verfahren zur Herstellung des erfindungsgemäßen Wirkstoffsystems vorgeschlagen, wobei in einem ersten Verfahrensschritt der Wirkstoffkomplex aus wenigstens einer in der Natur vorkommenden, nicht-synthetischen Nährstoffquelle ausgebildet wird.

Durch die Verwendung einer in der Natur vorkommenden nicht-synthetischen Nährstoffquelle wird das Herstellungsverfahren des Wirkstoffsystems vorteilhafterweise vereinfacht, da zum Einen die aufwendige Synthese einzelner Wirkstoffe als Komponenten des Wirkstoffsystems vollständig entfällt und zum Anderen, weil auf eine aufwendige Mischung der Einzelnährstoffe vollständig verzichtet werden kann.

Als natürlich vorkommende Nährstoffquelle zur Ausbildung des Wirkstoffkomplexes wird gemäß einem bevorzugten Merkmal der Erfindung Eiklar verwendet. Besonders bevorzugt ist hierbei die Verwendung von Eiklar aus Hühnereiern.

Das Eiklar kann hierbei in nativer Form vorliegen, wodurch der Anteil des Wirkstoffträgers durch den im Eiklar enthaltenen Wassergehalt verringert werden kann, was vorteilhafterweise den Herstellungsprozess vereinfacht.

Gemäß einer alternativen Ausgestaltung der Erfindung wird das Eiklar in getrockneter Form, insbesondere als Pulver oder Granulat, verwendet. Dies ist insbesondere hinsichtlich Haltbarkeits-, Lagerhaltungs- und Hygieneaspekten von Vorteil.

Gemäß einer bevorzugten Ausgestaltung der Erfindung wird der Wirkstoffkomplex im ersten Verfahrensschritt aus wenigstens Eiklar und Kieselerde ausgebildet. Vorzugsweise werden Eiklar und Kieselerde in einem Massenverhältnis von 3:1 bis 1:1, besonders bevorzugt 1:1, zur Ausbildung des Wirkstoffkomplexes eingesetzt. Es hat sich gezeigt, dass diese Massenverhältnisse nicht nur besonders vorteilhaft hinsichtlich der Faltenreduktion sind, sondern auch den Wirkstofftransport durch die Epidermis beschleunigen. Bevorzugterweise werden beide Komponenten in getrockneter Form verwendet und miteinander, insbesondere in einem Mischer, vermischt, bis sich ein im Wesentlichen homogenes Gemenge gebildet hat. Es ist ferner besonders bevorzugt weitere Komponenten, wie insbesondere Ubichinon im ersten Verfahrensschritt dem Eiklar und/oder der Mischung aus Eiklar und Kieselerde zur Erweiterung des Wirkstoffkomplexes beizumischen.

Erfindungsgemäß wird der Wirkstoffkomplex in einem zweiten Verfahrensschritt mit dem Wirkstoffträger gemischt. Vorzugsweise wird der Wirkstoffträger dem im ersten Verfahrensschritt gebildeten Wirkstoffkomplex im Mischer zugegeben und bis zur Homogenität gemischt. Der Wirkstoffträger ist hierbei vorzugsweise flüssig oder viskos ausgebildet. Als flüssiger Wirkstoffträger eignet sich insbesondere Wasser, da es leicht zugänglich und kostengünstig ist und darüber hinaus als Feuchtigkeitsspender für die Haut fungiert. Als viskoser Wirkstoffträger ist bevorzugt der Einsatz von dermatologisch unbedenklichen Gelen vorgesehen. Vorzugsweise können dem Wirkstoffträger weitere Inhaltstoffe, wie insbesondere Transportmoleküle, wie insbesondere cyclische Oligosaccharide, Micell- und/oder Liposombildner zugemischt werden. Hierbei ist der Einsatz von cyclischen Oligosacchariden, wie insbesondere Cyclodextrinen besonders bevorzugt.

Gemäß einem bevorzugten Merkmal der Erfindung werden der Wirkstoffkomplex und der Wirkstoffträger im Massenverhältnis von 1:1 bis 1:10, vorzugsweise 1:2 bis 1:5, besonders bevorzugt 1:3 bis 1:4 gemischt. Durch die Mischung im vorgenannten Verhältnis, ist das Wirkstoffsystem hinsichtlich seiner Viskosität, und insbesondere seiner damit zusammenhängenden ortsgenauen Anwendbarkeit, und seinem Wirkstoffgehalt optimiert, wodurch vorteilhafterweise die faltenreduzierende Wirkung des Wirkstoffsystems insgesamt verbessert wird.

Es ist ferner eine **Aufgabe** der Erfindung, ein kosmetisches Verfahren bereitzustellen, welches Faltenbildung nachhaltig, langfristig und auf natürlichem Wege mindert.

Zur **Lösung** dieser Aufgabe wird ein kosmetisches Verfahren zur Faltenreduzierung vorgeschlagen, bei welchem das erfindungsgemäße Wirkstoffsystem im Zuge einer Einzelanwendung auf die zu behandelnden Teile der Haut aufgebracht wird. Das Wirkstoffsystem durchdringt dabei zumindest teilweise die Epidermis und gibt seine Wirkstoffe an die Fibroblasten der Dermis ab. Die Einwirkzeit des Wirkstoffsystems beträgt vorzugsweise zwischen 5 und 45 min, vorzugsweise zwischen 15 und 30 min. Etwaige auf der Haut verbliebene Reste des Wirkstoffsystems können vorzugsweise durch Aufschwemmung mit einer Flüssigkeit, vorzugsweise Wasser, wieder in Lösung gebracht, bzw. emulgiert werden, wodurch die faltenreduzierende Wirkung durch erhöhte Aufnahme des Wirkstoffsystems verbessert wird.

Es ist gemäß einem bevorzugten Merkmal der Erfindung vorgesehen, dass die zu behandelnde Region der Haut vor der Einzelanwendung mit dem Wirkstoffsystem vorbehandelt wird. Die Vorbehandlung kann vorzugsweise aus einer Reinigung der Haut, Öffnung der Poren, insbesondere durch ein Peeling, und/oder aus der Auftragung einer hochdosierten Nährstofflösung bestehen. Die Nährstofflösung kann bevorzugt Nährstoffe aufweisen, die im Wirkstoffkomplex nicht oder in geringer Konzentration enthalten sind. Vorzugsweise sind dies Nährstoffe, die ebenfalls die Proteinbiosynthese und/oder die ATP-Bildungsprozesse der Atmungskette fördern und/oder als Anti-Oxidant wirken. Insbesondere zählen hierzu Vitamine, insbesondere B-Komplex Vitamine und Vitamin E, Biotin, Carnitin, Phosphor, Natrium, Kalium, Calcium und Magnesium. Es hat sich überraschenderweise gezeigt, dass das Wirkstoffsystem die Nährstoffe, die der Nährstofflösung entstammen durch die Epidermis transportieren. Der kombinierte Einsatz der Nähstofflösung und dem Wirkstoffsystem trägt somit in synergetischer Weise zu einer weiteren Verbesserung der Faltenreduktion bei.

Erfindungsgemäß wird eine Mehrzahl von Einzelanwendungen vorgenommen, um den Nährstoffmangel der Haut nachhaltig zu beheben eine langfristige Faltenreduktion zu erreichen. Der Zeitpunkt der jeweiligen Einzelanwendung wird hierbei über eine individuell verstellbare Anwendungssequenz bestimmt. Individuell erstellbar im Sinne der Erfindung bezeichnet hierbei ein Verfahren, bei dem in Abhängigkeit der Ausprägung des Faltenbildes der zu behandelnden Hautstelle, insbesondere durch Messung und Auswertung der Faltenverteilung, der Faltendichte sowie der Faltentiefe eine Anwendungssequenz im Sinne eines Behandlungsplans erstellt wird. Die erfindungsgemäße Anwendungssequenz gliedert sich in wenigstens zwei chronologisch aufeinander folgende Anwendungsphasen, in denen die Einzelanwendungen mit einer für die Anwendungsphase spezifischen Einzelanwendungsfrequenz durchgeführt werden. Erfindungsgemäß sind die Einzelanwendungsfrequenzen der einzelnen Anwendungsphasen voneinander verschieden.

Es hat sich vorteilhafterweise gezeigt, dass das erfindungsgemäße Verfahren das behandelte Faltenbild messbar, sichtbar und langfristig reduziert, indem es den Nährstoffmangel der Haut nachhaltig beseitigt und die Zellaktivität anregt. Es werden hierdurch nicht nur äußerlich Falten reduziert, sondern die Haut insgesamt revitalisiert, was insgesamt zu einem jungen und frischen Erscheinungsbild führt.

Gemäß einem bevorzugten Merkmal der Erfindung ist es vorgesehen, dass den einzelnen, chronologisch auf die erste Anwendungsphase folgenden Anwendungsphasen jeweils eine im Vergleich mit der Einzelanwendungsfrequenz der vorhergehenden Anwendungsphase verringerte Einzelanwendungsfrequenz zugewiesen wird. Hierdurch werden in der ersten Zeit der Behandlung häufiger Einzelanwendungen durchgeführt als im späteren Verlauf Behandlung. Vorteilhafterweise wird ein initialer, gravierender Nährstoffmangel der Haut damit wirkungsvoll beseitigt. Ist dieser initiale Mangel behoben, kann die Häufigkeit der Einzelbehandlungen reduziert werden, da nur noch die sich aus dem physiologischen Verbrauch von Nährstoffen ergebenden Mängel ausgeglichen werden müssen. Diese bevorzugte Ausgestaltung der Erfindung verbessert die faltenreduzierende Wirkung des Verfahrens insbesondere zu Beginn und verringert den Ressourcenverbrauch des Verfahrens insbesondere gegen Ende des Verfahrens, wodurch das erfindungsgemäße Verfahren vorteilhafterweise insgesamt verbessert wird.

Gemäß einer bevorzugten Ausgestaltung der Erfindung wird die Anwendungssequenz aus wenigstens drei Anwendungsphasen gebildet. Hierdurch lässt sich eine vergleichsweise feinere Abstimmung der Anwendungssequenz an die Ausprägung des zu behandelnden Faltenbildes und eine vergleichsweise differenziertere Behebung des Nährstoffmangels erreichen, wodurch eine weiter verbesserte Reduktion des Faltenbildes ermöglicht wird. Vorzugsweise werden die Einzelanwendungen in der ersten Anwendungsphase mit einer Einzelanwendungsfrequenz zwischen 1/12h und 1/60h, bevorzugt zwischen 1/24h und 1/48h durchgeführt. Ferner ist es bevorzugt, die Einzelanwendungen in der zweiten Anwendungsphase mit einer Einzelanwendungsfrequenz zwischen 1/48h und 1/120h, bevorzugt zwischen 1/72h und 1/96h durchgeführt. Es ist hierbei ferner bevorzugt, die Einzelanwendungen in der dritten Anwendungsphase mit einer Einzelanwendungsfrequenz zwischen 1/144h und 1/240h, bevorzugt zwischen 1/168h und 1/192 h durchgeführt.

Die Erfindung führt daher durch synergetische Wirkung seiner Merkmale zu einer nachhaltigen und langfristigen Faltenreduzierung der Haut.

Die folgenden Ausführungsbeispiele der Erfindung dienen lediglich der Veranschaulichung der Erfindung sind keinesfalls beschränkend zu verstehen, sondern dienen lediglich der Veranschaulichung der Erfindung.

### Herstellungsverfahren

Zur Herstellung des Wirkstoffsystems im Sinne der Erfindung werden zunächst Eiklarpulver und Kieselerdepulver in einem Massenverhältnis von 1:1 einer Mischeinrichtung zugeführt. Dort werden beide Komponenten bei Raumtemperatur solange gemischt, bis ein optisches homogenes Gemenge vorliegt. Besagtes Gemenge bildet hierbei den erfindungsgemäßen Wirkstoffkomplex. Durch die Verwendung ausschließlich natürlich vorkommender nicht-synthetischer Wirkstoffquellen ist in vorteilhafterweise sowohl das Herstellungsverfahren vereinfacht, als auch die Wirksamkeit des Wirkstoffsystems als Ganzes verbessert.

Im zweiten Verfahrensschritt werden den im Mischer befindlichen Wirkstoffkomplex nun 3,5 Massenäquivalente (bezogen auf die Einwaage des Wirkstoffkomplexes) eines Wirkstoffträgers, vorliegend ein Cyclodextrine aufweisendes Gel zugegeben. Diese Mischung wird nun solange bei Raumtemperatur gerührt, bis ein optisches homogenes Wirkstoffsystem in der gewünschten Viskosität vorliegt.

### Kosmetisches Verfahren

Zur erfolgreichen Anwendung des kosmetischen Verfahrens zur Faltenreduzierung wird zunächst die Ausprägung des Faltenbildes an der zu behandelnden Stelle detailliert analysiert, indem Faltenverteilung, Faltendichte und Faltentiefe gemessen werden. Vorliegend wird zu Anschauungszwecken von einem mittelschweren Faltenbild ausgegangen. Um eine langfristige und nachhaltige Faltenreduzierung zu erreichen wird eine Anwendungssequenz bestehend aus drei, chronologisch aufeinanderfolgenden Anwendungsphasen erstellt, wobei den drei Anwendungsphasen jeweils eine Dauer von einem Monat zugewiesen wird. Im ersten Monat werden die Einzelbehandlungen mit einer vergleichsweise hohen Frequenz durchgeführt. Im vorliegenden Fall wird in der ersten Anwendungsphase alle 48 Stunden eine Einzelanwendung durchgeführt.

Eine Einzelbehandlung besteht wenigstens aus dem topischen Auftragen des erfindungsgemäßen Wirkstoffsystems auf die zu behandelnde Hautstelle. Im vorliegenden Ausführungsbeispiel besteht die Einzelanwendung vorzugsweise aus vier Behandlungsschritten. Im ersten Behandlungsschritt wird die Haut mit einer Seifenlösung, vorzugsweise Tensidlösung, von Verunreinigungen, wie insbesondere Schweiß und Schmutzpartikeln gereinigt. Hierdurch wird insbesondere die gleichmäßige Aufnahme des Wirkstoffsystems durch die Haut verbessert, da die vorgenannten Verunreinigungen eine Aufnahme des Wirkstoffsystems lokal verhindern. Im zweiten Behandlungsschritt werden abgestorbene Hautreste entfernt und die Hautporen geöffnet. Dies geschieht vorliegend durch ein Hautpeeling. Durch Entfernung der abgestorbenen Hautschichten sowie der Öffnung der Poren wird die Durchlässigkeit der Haut insgesamt verbessert, wodurch die Aufnahme des Wirkstoffsystems durch die Epidermis beschleunigt wird. Im dritten Behandlungsschritt wird die Haut dann mit einer hochdosierten Nährstofflösung behandelt. Hierzu wird besagte Nährstofflösung auf die zu behandelnde Hautstelle aufgebracht. Die Nährstofflösung besteht vorwiegend aus Vitamin A, Vitamin C, Vitamin B, Vitamin B1, Vitamin B5, Vitamin B6, Vitamin B12, Vitamin E, Biotin, Carnitin, Phosphor, Natrium, Kalium, Calcium und Magnesium. Im vierten Behandlungsschritt wird das erfindungsgemäße Wirkstoffsystem nun auf den sich auf der zu behandelnden Hautstelle befindlichen Nährstofffilm aufgebracht. Das erfindungsgemäße Wirkstoffsystem sorgt vorteilhafterweise nicht nur für den Transport der eigenen Nährstoffe, sondern auch für den Transport der in der Nährstofflösung befindlichen Nährstoffe durch die Epidermis. Nach einer Einwirkzeit des Wirkstoffsystems von 20 Minuten, bildet sich auf der Haut ein pergamentartiger Nährstofffilm, welcher vorteilhafterweise eine Abgabe von Feuchtigkeit an die Umgebung durch die Hautporen während der Behandlung verhindert und die Aufnahme der Wirkstoffe sowie die Aufnahme von Feuchtigkeit durch die Haut verbessert. Der Nährstofffilm wird nun vorliegend mit Wasser aufgeschwämmt, wodurch etwaige noch nicht durch die Epidermis transportierte Nährstoffe der Dermis zugeführt werden. Die Einwirkzeit nach der Aufschwämmung beträgt vorliegend fünf bis zehn Minuten, vorzugsweise fünf Minuten. Ein nach der vorbenannten Einwirkzeit etwaiger immer noch verbleibender Nährstofffilm kann so oft wie möglich erneut aufgeschwämmt werden, um die Nährstoffaufnahme weiter zu verbessern oder entfernt werden, um die Behandlungszeit zu verkürzen.

Durch die hohe Anwendungsfrequenz der Einzelanwendungen in der ersten Anwendungsphase wird vorteilhafterweise ein initialer, besonders gravierender Nährstoffmangel in der Haut behoben.

In der zweiten Anwendungsphase wird die Anwendungsfrequenz der Einzelanwendungen reduziert, um dem verringerten Nährstoffbedarf der Haut Rechnung zu tragen. Vorliegend werden in der zweiten Anwendungsphase Einzelanwendungen mit einer Frequenz von drei Tagen (72 Stunden) durchgeführt.

Zu Beginn der dritten Anwendungsphase ist die Nährstoffversorgung der Hautzellen nun soweit verbessert, dass die Einzelanwendungsfrequenz weiter gesenkt werden kann. Vorliegend werden in der dritten Anwendungsphase Einzelanwendungen mit einer Frequenz von acht Tagen (192 Stunden) durchgeführt. Das erfindungsgemäße kosmetische Verfahren gemäß diesem Ausführungsbeispiel sorgt vorteilhafterweise für eine langfristige und nachhaltige Reduzierung des Faltenbildes an der behandelten Hautstelle, durch Versorgung der für die Bioproteinsynthese der Bindegewebsproteine und der für die Energiegewinnung verantwortlichen Hautzellen mit den hierfür notwendigen Nährstoffen.

## Patentansprüche

1. Wirkstoffsystem für kosmetische Zwecke zur topischen Anwendung auf der Haut, aufweisend einen Wirkstoffträger und einen Wirkstoffkomplex, **dadurch gekennzeichnet, dass** der Wirkstoffkomplex wenigstens eine in der Natur vorkommende, nicht synthetische Nährstoffquelle enthält.

2. Wirkstoffsystem nach Anspruch 1, **dadurch gekennzeichnet, dass** die Nährstoffquelle aus Eiklar gebildet ist.

3. Wirkstoffsystem nach einem der vorhergehenden Ansprüche, **dadurch gekennzeichnet, dass** der Wirkstoffkomplex Kieselerde enthält.

4. Wirkstoffsystem nacheinem der Ansprüche 1 oder 2, **dadurch gekennzeichnet, dass** der Wirkstoffkomplex Ubichinon-10 enthält.

5. Wirkstoffsystem nach einem der vorhergehenden Ansprüche, **dadurch gekennzeichnet, dass** der Wirkstoffträger cyclische Oligosaccharide enthält.

6. Verfahren zur Herstellung des Wirkstoffsystems gemäß den Ansprüchen 1 bis 5, wobei in einem ersten Verfahrensschritt der Wirkstoffkomplex aus wenigstens einer in der Natur vorkommenden, nicht-synthetischen Nährstoffquelle ausgebildet wird, und wobei in einem zweiten Verfahrensschritt der Wirkstoffkomplex mit dem Wirkstoffträger gemischt wird.

7. Verfahren nach Anspruch 6, **dadurch gekennzeichnet, dass** im ersten Verfahrensschritt der Wirkstoffkomplex aus wenigstens Eiklar und Kieselerde ausgebildet wird.

8. Kosmetisches Verfahren zur Faltenreduzierung, wobei das Wirkstoffsystem gemäß den Ansprüchen 1 bis 5 bei einer Einzelanwendung auf die Haut aufgebracht wird, wobei eine Mehrzahl von Einzelanwendungen durchgeführt wird, wobei der Zeitpunkt der jeweiligen Einzelanwendung durch eine individuell erstellbare Anwendungssequenz bestimmt wird, wobei die Anwendungssequenz aus wenigstens zwei chronologisch aufeinander folgenden Anwendungsphasen mit anwendungsphasenspezifischen Einzelanwendungsfrequenzen gebildet wird, wobei den Anwendungsphasen voneinander verschiedene Einzelanwendungsfrequenzen zugewiesen werden.

9. Kosmetisches Verfahren nach Anspruch 8, **dadurch gekennzeichnet, dass** den einzelnen, chronologisch auf die erste Anwendungsphase folgenden Anwendungsphasen jeweils eine im Vergleich mit der Einzelanwendungsfrequenz der vorhergehenden Anwendungsphase verringerte Einzelanwendungsfrequenz zugewiesen wird.

10. Kosmetisches Verfahren nach einem der Ansprüche 8 oder 9, **dadurch gekennzeichnet, dass** die Anwendungssequenz aus wenigstens drei Anwendungsphasen gebildet wird.
